# EUROPEAN PATENT APPLICATION

(11) **EP 4 295 919 A1**
(43) Date of publication of application: **27.12.2023**
(21) Application number: 22756230.3
(22) Date of filing: 16.02.2022
(51) Int. Cl.: A61Q 1/00, A61K 8/04, A61K 8/19, A61K 8/41

(54) **PRUSSIAN BLUE DISPERSION FOR COSMETICS, AND COSMETICS INCLUDING SAME**

(30) Priority: 18.02.2021 JP 2021024272; 02.09.2021 JP 2021143153
(71) Applicant: MITSUBISHI PENCIL COMPANY, LIMITED, Tokyo 140-8537 (JP)
(72) Inventor: INOUE Kensuke, Fujioka-shi, Gunma 375-8501 (JP); HAYAKAWA Takayuki, Tokyo 140-8537 (JP); SAKUMA Satoshi, Fujioka-shi, Gunma 375-8501 (JP)
(74) Representative: Vossius & Partner Patentanwälte Rechtsanwälte mbB
(86) International application number: PCT/JP2022/006189
(87) International publication number: WO 2022/176912

(57) **Abstract**

Provided is a Prussian blue dispersion for cosmetics excellent in dispersion stability, color-developing properties (high chroma), and storage stability, and a cosmetic in which the Prussian blue dispersion is blended, the cosmetic being suitable for makeup cosmetics such as eyeshadows, eyeliners, eyebrow pencil, and mascaras. Further provided is a green liquid cosmetic excellent in green color developing properties (high chroma) and dispersion stability, the cosmetic being suitable for makeup cosmetics such as eyeshadows, eyeliners, eyebrow pencil, and mascaras. The Prussian blue dispersion for cosmetics of the present disclosure includes at least Prussian blue, a water-soluble basic substance, and water. The cosmetic of the present invention is formed by blending the Prussian blue dispersion. Further, the green liquid cosmetic is obtained by blending the Prussian blue dispersion with a red iron oxide dispersion of red color having a specific property.

## Description

### Technical Field

The present specification relates to a Prussian blue dispersion for cosmetics excellent in dispersion stability, color-developing properties (high chroma), and storage stability, and a cosmetic in which the Prussian blue dispersion is blended, the cosmetic being suitable for makeup cosmetics such as an eyeshadow, an eyeliner, an eyebrow pencil, and a mascara.

### Background Art

Prussian blue has typically been widely used as a coloring material and mixed with an extender pigment for coloring cosmetics, paints, writing inks, printing inks, resins, and the like (see, for example, Non-Patent Document 1).

The Prussian blue contains ferric ferrocyanide as a main component and its common chemical structure is represented by MFe[Fe(CN)₆], M = K, Na, NH₄. Commercially available products are mainly ferric ammonium ferrocyanide, having deep blue color unique to Prussian blue and having a high coloring strength as an inorganic pigment, but it has a problem that its stable pH is as low as 4 to 6 and is unstable under alkaline conditions (see, for example, Patent Documents 1 and 2 and Non-Patent Document 2).

In the alkali conditions, the color may be faded, and when the Prussian blue is used in combination with an alkaline extender pigment such as calcium carbonate or alumina, the color may be faded or lost. In addition, when Prussian blue is reduced, the blue color may be lost, or when Prussian blue is kneaded with a vehicle which is easily oxidized then sealed, Prussian blue may act as an oxidizing agent and the Prussian blue itself may be reduced and faded.

On the other hand, in cosmetics and the like, Prussian blue has a higher coloring strength than ultramarine blue, which is a blue-based pigment, provides a blue color tone with high chroma, and provides a green color with high chroma when used in combination with, for example, a legal yellow coloring matter. Thus, there is a high need for using Prussian blue as a blending component of cosmetics, but the use of Prussian blue may be limited because of the above problems.

For example, a surface-treated powder obtained by treating a surface-treated Prussian blue-coated powder with either or both of α-oxycarboxylic acid and a coating-forming substance (see Patent Document 1) is known as a solution to the problems of Prussian blue. A pigment dispersion for cosmetics containing black iron oxide, red iron oxide, and Prussian blue (see Patent Document 2) is known as a cosmetic containing Prussian blue.

However, the above Patent Document 1 has problems that it does not provides the inherent color-developing properties of Prussian blue and it is insufficient in dispersion stability. The above Patent Document 2 produces a dispersion of mixed black obtained by simply blending three components of black iron oxide, red iron oxide, and Prussian blue, in which the above problems of Prussian blue is not mentioned. In Patent Document 2, the Prussian blue is not prepared as a dispersion in advance and used, where dispersion stability is maintained. These documents are different from the present invention in object of invention, technical ideas, and so forth.

### Citation List

### Patent Document

Non-Patent Document 1 : New Illustrated Powder Physical Properties (third edition) (third edition, first printing, third issue, June 30, 2004, p. 343)
Non-Patent Document 2 : "Pigment Introduction Course Text", Japan Society of Colour Material, 1970, pp. 114 to 118)

Patent Document 1: JP 2004-91645 A (Claims, Description, etc.)
Patent Document 2: JP 2003-231614 A (Claims, Description, etc.)

### Summary of Invention

### Technical Problem

The present disclosure has been made in view of the above-described problems, current state of the related art, and an object of the present disclosure is to provide a Prussian blue dispersion for cosmetics excellent in dispersion stability, color-developing properties (high chroma), and storage stability, and a cosmetic in which the Prussian blue dispersion is blended, the cosmetic being suitable for makeup cosmetics such as an eyeshadow, an eyeliner, an eyebrow pencil, and a mascara. Another object of the present disclosure is to provide a green liquid cosmetic excellent in green color developing properties (high chroma), dispersion stability, and weather resistance, the green liquid cosmetic being suitable for makeup cosmetics such as an eyeshadow, an eyeliner, an eyebrow pencil, a mascara, and the like.

### Solution to Problem

As a result of intensive studies in view of the problems in the above-described related art and the like, the inventors of the present invention have found that an objective Prussian blue dispersion for cosmetics and a cosmetic containing the Prussian blue dispersion can be obtained by containing at least Prussian blue, a specific component, and water. More surprisingly, they found that a green cosmetic can be obtained as a part of a cosmetic in which the Prussian blue dispersion is blended. And thus the present disclosure is completed.

That is, a Prussian blue dispersion for cosmetics of the present disclosure contains at least Prussian blue, a water-soluble basic substance, and water.

The Prussian blue dispersion preferably contains the water-soluble basic substance in a range of 0.025 to 0.25 when a content of the Prussian blue is defined as 1 in terms of mass ratio.

The water-soluble basic substance is preferably at least one type selected from ammonia, a compound having an amino group, or an alkali metal hydroxide.

The Prussian blue dispersion preferably has a viscosity of 50 mPa·s or less at a shear rate of 383 s⁻¹ in viscosity measurement at 25°C with a cone-plate type viscosimeter.

The Prussian blue in the Prussian blue dispersion for cosmetics preferably has an average particle size of 200 nm or less as determined by cumulant method analysis in a scattering intensity distribution. The Prussian blue dispersion preferably further contains a dispersant. A cosmetic of the present disclosure is a cosmetic formed by blending the Prussian blue dispersion for cosmetics.

In the cosmetic of the present disclosure, preferably an iron oxide pigment dispersion is further blended in addition to the Prussian blue dispersion for cosmetics.

A green liquid cosmetic of the present disclosure is a green liquid cosmetic including a Prussian blue dispersion for cosmetics and an iron oxide pigment dispersion, wherein the Prussian blue dispersion for cosmetics is a Prussian blue dispersion for cosmetics (A) containing at least the Prussian blue, the water-soluble basic substance, and water, and an amount of the water-soluble basic substance is 0.05 to 0.13 when a content of the Prussian blue is defined as 1 in terms of mass ratio; the iron oxide pigment dispersion is a red iron oxide dispersion for cosmetics (B) containing red iron oxide, an organic acid having a molecular weight of 300 or less, and water, and the red iron oxide has an average particle size of 100 nm or less as determined by cumulant method analysis in a scattering intensity distribution.

In the green liquid cosmetic, the Prussian blue in the Prussian blue dispersion for cosmetics (A) preferably has an average particle size of 130 nm or less as determined by cumulant method analysis in a scattering intensity distribution.

In the green liquid cosmetic, the organic acid having a molecular weight of 300 or less in the red iron oxide dispersion for cosmetics (B) is preferably selected from Group A below:
Group A: glycolic acid, lactic acid, tartronic acid, glyceric acid, hydroxybutyric acid, 2-hydroxybutyric acid, 3-hydroxybutyric acid, γ-hydroxybutyric acid, malic acid, tartaric acid, citramalic acid, citric acid, isocitric acid, leucinic acid, mevalonic acid, pantoic acid, ricinoleic acid, ricinelaidic acid, cerebronic acid, quinic acid, shikimic acid

In the green liquid cosmetic, the organic acid having a molecular weight of 300 or less in the red iron oxide dispersion for cosmetics (B) is preferably citric acid.

### Advantageous Effects of Invention

The present disclosure provides a Prussian blue dispersion for cosmetics excellent in dispersion stability, color-developing properties (high chroma), and storage stability, and a cosmetic in which the Prussian blue dispersion is blended, the cosmetic being suitable for makeup cosmetics such as an eyeshadow, an eyeliner, an eyebrow pencil, and a mascara. Further, the present disclosure provides a green liquid cosmetic excellent in green color developing properties (high chroma) and dispersion stability, the green liquid cosmetic being suitable for makeup cosmetics such as an eyeshadow, an eyeliner, an eyebrow pencil, and a mascara.

The object and effects of the present disclosure can be recognized and obtained specifically using the components and combinations indicated in the claims. Both of general explanation described above and detailed explanation described below are exemplification and explanation and do not limit the present invention described in Claims.

### Brief Description of Drawings

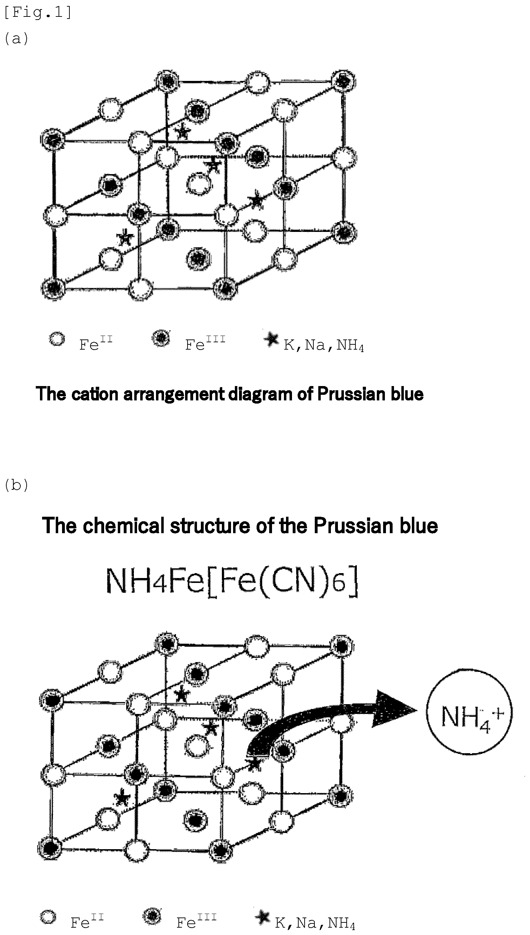

FIG. 1(a) illustrates an example of a cation arrangement diagram of Prussian blue, and (b) is an explanatory diagram for explaining an outline of a mechanism for maintaining dispersion stability and the like of the Prussian blue of the present disclosure.

### Description of Embodiments

Embodiments of the present disclosure will be described below in detail. However, note that the technical scope of the present disclosure is not limited to the embodiments described below and includes the invention described in Claims and equivalents of the invention.

### <Prussian blue dispersion for cosmetics>

The Prussian blue dispersion for cosmetics of the present disclosure contains at least Prussian blue, a water-soluble basic substance, and water.

The chemical structure of the Prussian blue used in the present disclosure can be commonly represented by MKFe[Fe(CN)₆], wherein M is any one of K, Na, or NH₄. Iron ions include divalent iron (Fe^{II}) and trivalent iron (Fe^{III}). FIG. 1 is a cation arrangement diagram of the Prussian blue.

The Prussian blue used in the present disclosure can be used without particular limitation as long as it is Prussian blue commonly used in cosmetic products, and it preferably has an average particle size of 200 nm or less, and it is preferably ferric ammonium ferrocyanide in which M is NH₄ from the viewpoint of availability or the like. The Prussian blue may be surface-modified Prussian blue, and commercially available products thereof may be used.

Examples of the commercially available product include MILORI BLUE FX-6940 and FX-9050 available from Dainichiseika Color & Chemicals Mfg. Co., Ltd., Iron Blue SC available from Daito Chemical Industry Co., Ltd., and C38-7710 Softex Iron Blue available from Sun Chemical Company Ltd. As described later, the "average particle size" in the present disclosure refers to an average particle size obtained by cumulant method analysis in a scattering intensity distribution.

The Prussian blue dispersion has a stable pH as low as 4 to 6 and has a problem that it is unstable under alkaline conditions, and problems in productivity and dispersion stability in the production of the dispersion. However, the dispersion having the formulation properties of the present disclosure can solve the problems.

The content of the Prussian blue to be used is preferably 5 to 50 mass% (hereinafter, "mass%" is simply referred to as "%"), and more preferably 10 to 30% with respect to the total amount of the Prussian blue dispersion for cosmetics, from the viewpoint of stability after dispersion and convenience in the production of cosmetics.

Setting the content of this Prussian blue to 5% or greater leads to excellent productivity and coloration properties when the Prussian blue dispersion is added to cosmetics. Setting the content to 50% or less leads to even better dispersibility and stability over time.

According to the findings of the inventors of the present invention, as illustrated in FIG. 1(b), when dispersion of Prussian blue progresses, NH₄⁺ is eliminated from the Prussian blue, H⁺ is generated from NH₄⁺, the pH of the dispersion tends to decrease, and the absolute value of the zeta potential of the Prussian blue at a lower pH tends to decrease. By using a water-soluble basic substance serving as an alkaline agent for capturing H⁺, the surface potential is maintained, and dispersion stability and finer particle sizes are maintained.

The water-soluble basic substance used in the present disclosure is used for this purpose.

Specific examples of the water-soluble basic substance that can be used include at least one type of (alone or combination: a mixture of two types or more) alkali metal hydroxides such as sodium hydroxide and potassium hydroxide, alkali metal carbonates such as potassium carbonate and sodium carbonate, ammonia, amines such as monomethylamine, dimethylamine, triethylamine, monoethylamine, diethylamine, ethylenediamine, monoethanolamine, diethanolamine, and triethanolamine, which are compounds having an amino group, basic amino acids such as aminomethylpropanol(2-amino-2-methyl-1-propanol), trishydroxymethylaminomethane, arginine, and lysine, aminoethylpropanolamine, aminomethylpropanediol, aminoethylpropanediol, or the like.

In particular, it is preferable to use aminomethylpropanol or potassium hydroxide from the viewpoint of preventing change in color and aggregation of Prussian blue at most and further realizing the effect of the present invention.

The content of the water-soluble basic substance to be used is such that the water-soluble basic substance is contained preferably in a range of 0.025 to 0.25 in terms of mass ratio, further preferably in a range of 0.05 to 0.13 in terms of mass ratio, and particularly preferably in a range of 0.05 to 0.10 in terms of mass ratio when the mass of the Prussian blue is defined as 1, from the view point of dispersibility of Prussian blue and preventing the viscosity from increasing.

When the mass ratio of the water-soluble basic substance is less than 0.025, gelation occurs at the time of dispersion, and the effect of the present disclosure cannot be exhibited. On the other hand, when the mass ratio is more than 0.25, the viscosity after dispersion is too high, and the degree of freedom in ink design decreases.

The Prussian blue dispersion for cosmetics of the present disclosure preferably further contains a dispersant.

The dispersant that can be used in the present disclosure is contained from the viewpoints of adjusting the viscosity and further improving the dispersion stability of the Prussian blue, and examples thereof include at least one type of a surfactant or a polymer dispersant. As the surfactant, any of commonly used surfactants such as anionic surfactants, nonionic surfactants, cationic surfactants, amphoteric surfactants, and polymer surfactants may be used, and, two or more thereof may be mixed and used.

Of these surfactants, nonionic surfactants are preferable from the viewpoints of obtaining more stable dispersibility of Prussian blue, low irritation, and elimination of influence on the dispersion state. Examples of the nonionic surfactant that is preferably used include polyoxyethylene alkyl ethers such as laureths (polyoxyethylene lauryl ether), ceteths (polyoxyethylene cetyl ether), steareths (polyoxyethylene stearyl ether), and beheneths (polyoxyethylene behenyl ether), and these can be used alone or in combination of two or more. Of these, beheneth-30, ceteth-20, and laureth-21 are preferably used from the viewpoint of their molecular structure and hydrophilicity.

As the polymer dispersant, for example, one or more selected from polyaspartic acid, polyglutamic acid, a styrene-acrylic acid copolymer, a styrene-methacrylic acid copolymer, and a styrene-α-methylstyrene-acrylic acid copolymer, or one or more salts thereof may be contained. Examples of the salts include alkali salts such as sodium, potassium, and lithium; ammonium salts; and alkanolamine salts such as mono-, di-, triethanolamine, and triisopropanolamine. Of these polymer dispersants, at least one type of polyaspartic acid, polyglutamic acid, a styrene-acrylic acid copolymer, a styrene-methacrylic acid copolymer, a styrene-α-methylstyrene-acrylic acid copolymer, or a salt thereof is particularly preferably used because of excellent dispersion stability. Particularly, sodium polyaspartate (Na) is preferred.

The content of these dispersants is preferably 0 to 5%, and more preferably 0 to 3% with respect to the entire (total amount of) liquid cosmetic.

When the content of the dispersant exceeds 5%, the stability of the cosmetic is adversely affected, which is not preferable.

The balance of the dispersion is adjusted with water (purified water, distilled water, ion exchanged water, pure water, tap water, etc.). The Prussian blue dispersion for cosmetics according to the present disclosure contains at least the Prussian blue, the water-soluble basic substance, and water. From the viewpoint of further improving dispersibility and dissolution stability of each component, a viscosity modifier, a chelating agent, a dispersant other than those described above, a pH modifier, and the like may be appropriately used as necessary.

The Prussian blue dispersion for cosmetics of the present disclosure can be produced by blending components including at least the Prussian blue, the water-soluble basic substance, the dispersant, and water in the ranges of the contents described above, uniformly stirring and mixing the components.

For example, a Prussian blue dispersion for cosmetics (A) can be prepared by stirring the Prussian blue, the water-soluble basic substance, the dispersant, and a solvent such as water with a general-purpose disperser or the like until they are uniformly mixed, further stirring the mixture with a disperser or the like such as a homomixer until the mixture is uniformly mixed, or mixing and dispersing the mixture with various stirrers or dispersion machines such as an ultrasonic disperser, a planetary mixer, a three roll mill, a ball mill, a bead mill, and a jet mill, in addition to a disperser.

These various stirrers and dispersion machines are optimally selected depending on the types and blending ratios of the components to be blended, the viscosity of the dispersion liquid obtained by stirring and mixing, and the like. The dispersion conditions are not particularly set, and the dispersion can be performed under known dispersion conditions (peripheral speed, time, etc.) . For example, when LMZ-4 (available from Ashizawa Finetech Ltd.) is used, the peripheral speed is 5 m/s or greater, and the time is 60 min or longer.

In the Prussian blue dispersion for cosmetics of the present disclosure, the viscosity at a shear rate 383 s⁻¹ as measured at 25°C with a cone-plate type viscosimeter is preferably 50 mPa·s or less, more preferably 10 mPa·s or less, further preferably 2 to 8.5 mPa·s, and particularly preferably 2 to 6 mPa·s from the viewpoint of appropriate viscosity, dispersion stability of the Prussian blue, improving blending properties to cosmetics, and improving outflow of a collector-type pen-shaped applicator.

The viscosity range (50 mPa·s or less) can be adjusted by suitably adjusting the amounts of the components to be used, the type and amount of the dispersant to be preferably used as described above, and the type and amount of a pH modifier.

In the Prussian blue dispersion for cosmetics used in the present disclosure, the average particle size of Prussian blue in a dispersed state is 200 nm or less, preferably 130 nm or less, and more preferably 80 to 130 nm as determined by cumulant method analysis in a scattering intensity distribution.

When the average particle size of the Prussian blue is 200 nm or less, preferably 130 nm or less, more stable dispersion and more vivid color development can be achieved. The lower limit of the average particle size is preferably as low as possible but is preferably 80 nm or greater in view of productivity, cost, and the like.

In the present disclosure, examples of the measurement device include FPAR-1000 (available from Otsuka Electronics Co., Ltd.) for a dynamic light scattering method, Microtrac (available from Nikkiso Co., Ltd.) for a laser diffraction/scattering method, and Mac-View (available from Mountech Co., Ltd.) for an imaging method.

The pH of the Prussian blue dispersion for cosmetics used in the present disclosure is preferably adjusted to 7.5 or less, and further preferably adjusted to 4.0 to 7.2 from the viewpoint of improving coloration properties, preventing skin irritancy, and dispersion stability.

In the present invention, the pH of the dispersion can be adjusted by using an acidic substance such as citric acid or a basic substance such as 2-amino-2-methyl-1-propanol.

Since the Prussian blue dispersion for cosmetics of the present disclosure configured as described above contains at least Prussian blue, a water-soluble basic substance, and water, the problems of a decrease in the absolute value of the zeta potential of Prussian blue, aggregation, and change in color can be solved. As the surface potential of Prussian blue can be maintained, and dispersion stability and finer particle sizes can be maintained, a Prussian blue dispersion for cosmetics, which is excellent in dispersion stability, color-developing properties (high chroma), and storage stability over time, can be obtained.

In addition, the obtained Prussian blue dispersion for cosmetics of the present disclosure can be developed into an applicator for water-based cosmetics and the like such as a direct-liquid eyeliner as a new dispersion technique of Prussian blue because a high-chroma blue color tone can be obtained, and because of improvement in finer particle sizes and dispersion stability. Further, because a pigment dispersion of high-chroma green or other different colors (increased variation in mixed color) or cosmetic can be obtained by using a dispersion of red iron oxide or a legal yellow coloring matter, it can be suitably used as a pigment-blending component for water-based cosmetics and the like.

The obtained Prussian blue dispersion for cosmetics is suitably used for applications such as cosmetics using Prussian blue and compositions for body markers. Preferred examples of the cosmetic applications include skincare cosmetics, makeup cosmetics, and nail cosmetic products, which include: makeup cosmetics such as foundations, eyeshadows, eyeliners, eyebrow pencils, mascaras, blushes, nail colors, nail treatments, various gel nails, and lipsticks; and hair colors. Examples of the compositions for body markers include body paints. The form of the product is not particularly limited, but it can be applied to aqueous products such as liquids, emulsions, creams, pastes, gels, mousses, and sprays, because it is a dispersion liquid (aqueous).

In particular, the Prussian blue dispersion for cosmetics of the present disclosure is preferably used for aqueous liquid cosmetics such as eye makeup cosmetics including eyeshadows, eyeliners, eyebrow pencils, and mascaras, and aqueous nail colors owing to its dispersion properties.

### Cosmetic

The cosmetic of the present disclosure is formed by blending the above-described Prussian blue dispersion for cosmetics. Various types of cosmetics can be prepared by blending the Prussian blue dispersion for cosmetics of the present disclosure with general-purpose blending components for each application of skin care cosmetics, makeup cosmetics, and nail cosmetic products.

For example, when an eyeliner is prepared as a cosmetic containing the Prussian blue dispersion for cosmetics, the cosmetic contains at least 1 to 60% of the Prussian blue dispersion for cosmetics, a solvent, a pH modifier, and 5 to 30% of a film-forming agent. However, other raw materials such as solvents, thickeners, various surfactants (POE alkyl ethers) other than those described above, preservatives (phenoxy ethers, parabens), ultraviolet absorbers, antioxidants, reduction inhibitors, chelating agents, oily components, fragrances, and animal and plant extracts can be contained in appropriate amounts as long as the effect of the present invention is not impaired.

In the present disclosure, the balance (remainder) of the cosmetic is adjusted with water (purified water, distilled water, ion exchanged water, pure water, tap water, etc.).

### Iron oxide pigment dispersion

In the cosmetic of the present disclosure, as a formulation example of a cosmetic containing the Prussian blue dispersion for cosmetics, naturally, a cosmetic of each color can be obtained by combining the dispersion with an iron oxide [yellow iron oxide, red iron oxide, and black iron oxide] pigment dispersion. For example, a dark blue cosmetic can be obtained with a black iron oxide pigment dispersion. In addition, as described above, a green cosmetic having high chroma can be obtained by combining the dispersion with a red iron oxide dispersion of yellow color. Further, a purple cosmetic can be obtained by combining the dispersion with a dispersion of ordinary red iron oxide dispersion of red color.

Examples of the iron oxide pigment dispersion used in the cosmetic of the present disclosure include a black iron oxide dispersion, a red iron oxide dispersion of yellow color, and an ordinary red iron oxide dispersion of red color.

In the cosmetic of the present disclosure, surprisingly, it has been found that a pigment dispersion or cosmetic of high-chroma green (increased variation in mixed color) can be obtained even by combining the dispersion with a red iron oxide dispersion for cosmetics having a specific property, particularly a red iron oxide dispersion of red color having a specific property, and the pigment dispersion or cosmetic can be suitably used as a pigment blending component of an aqueous cosmetic or the like. In particular, in the case of a green liquid cosmetic, the following formulation is preferable.

### Green liquid cosmetic

The green liquid cosmetic of the present disclosure includes at least the Prussian blue dispersion for cosmetics and a black iron oxide pigment dispersion, wherein the Prussian blue dispersion for cosmetics is a Prussian blue dispersion for cosmetics (A) containing at least Prussian blue, a water-soluble basic substance, and water, and an amount of the water-soluble basic substance is 0.05 to 0.13 when a content of the Prussian blue is defined as 1 in terms of mass ratio; the iron oxide pigment dispersion is a red iron oxide dispersion for cosmetics (B) containing red iron oxide, an organic acid having a molecular weight of 300 or less, and water, and the red iron oxide has an average particle size of 100 nm or less as determined by cumulant method analysis in a scattering intensity distribution.

In the green liquid cosmetic of the present disclosure, the Prussian blue dispersion for cosmetics to be used as the (A) may be the Prussian blue dispersion for cosmetics described in detail above, and the range of the content of the water-soluble basic substance is further limited to some extent, that is, the content of the water-soluble basic substance is used in a range of 0.05 to 0.13 when the content of Prussian blue in the Prussian blue dispersion for cosmetics is defined as 1 in terms of mass ratio, whereby a green liquid cosmetic containing a pigment dispersion of high-chroma green color (increased variation in mixed color) which has never existed before can be obtained.

In the green liquid cosmetic of the present disclosure, the average particle size of the Prussian blue to be used in the Prussian blue dispersion for cosmetics is further preferably 130 nm or less, particularly preferably 80 to 130 nm as determined by cumulant method analysis in a scattering intensity distribution. Further using Prussian blue having an average particle size in this range can makes it possible to obtain a green liquid cosmetic further excellent in coating properties and stability, the green liquid cosmetic containing a pigment dispersion of high-chroma green (increased variation in mixed color).

### <Red iron oxide dispersion for cosmetics (B)>

The red iron oxide dispersion for cosmetics (B) used in the green liquid cosmetic of the present disclosure is a red iron oxide dispersion for cosmetics of red color. The red iron oxide dispersion contains at least iron oxide, an organic acid having a molecular weight of 300 or less, and water, wherein the red iron oxide has an average particle size of 100 nm or less as determined by cumulant method analysis in a scattering intensity distribution. Surprisingly, the green liquid cosmetic of the present disclosure can be obtained by combining and mixing the Prussian blue dispersion for cosmetics having the above-described properties and the red iron oxide dispersion for cosmetics having such a red color development.

As the red iron oxide to be used, various known red iron oxides may be **used, and examples thereof include red iron oxides including red α**-Fe₂O₃ **(hematite), brown γ**-Fe₂O₃ (maghemite), black Fe₃O₄ (magnetite), yellow **α-FeOOH, and the like as main components, and in particular, red α**-Fe₂O₃ (hematite) is used in the present invention from the viewpoint of obtaining a green cosmetic.

The content of this **red iron oxide [α**-Fe₂O₃ (hematite) or the like] is preferably 10 to 50%, and more preferably 15 to 40% with respect to the total amount of the red iron oxide dispersion, from the viewpoint of stability after dispersion and convenience in the production of cosmetics.

**Setting the content of this red iron oxide [α-**Fe₂O₃ (hematite) or the like] to 10% or greater leads to excellent productivity and coloration properties when the dispersion is added to a green liquid cosmetic (mixed with Prussian blue dispersion) . Setting the content to 50% or less leads to better dispersibility and stability over time.

The organic acid having a molecular weight of 300 or less to be used improves the dispersion of the red iron oxide liquid, and examples thereof include at least one type selected from organic acids such as aliphatic hydroxy acids, aromatic hydroxy acids or derivatives thereof having a molecular weight of 300 or less.

Examples of the aliphatic hydroxy acid include glycolic acid, lactic acid, tartronic acid, glyceric acid, hydroxybutyric acid, 2-hydroxybutyric acid, 3-**hydroxybutyric acid, γ**-hydroxybutyric acid, malic acid, tartaric acid, citramalic acid, citric acid, isocitric acid, leucinic acid, mevalonic acid, pantoic acid, ricinoleic acid, ricinelaidic acid, cerebronic acid, quinic acid, and shikimic acid. Examples of the aromatic hydroxy acid and derivatives thereof include monohydroxybenzoic acid derivatives, salicylic acid, creosote acid [homosalicylic acid, hydroxy(methyl)benzoic acid], vanillic acid, syringic acid, dihydroxybenzoic acid derivatives, pyrocatechuic acid, resorcylic acid, protocatechuic acid, gentisic acid, olseric acid, trihydroxybenzoic acid derivatives, gallic acid, phenylacetic acid derivatives, mandelic acid, benzilic acid, atrolactic acid, cinnamic acid, hydrocinnamic acid derivatives, melitonic acid, phloretic acid, coumaric acid, umbellic acid, caffeic acid, ferulic acid, and sinapic acid.

Preferred are those selected from the above aliphatic hydroxy acids (Group A), and more preferred is citric acid from the viewpoint of safety as an eyeliner.

In the present disclosure, an acid having a molecular weight of more than 300 is not preferable because an aggregation tendency of red iron oxide or ultramarine increases.

The content of the organic acid having a molecular weight of 300 or less is preferably 0.01 to 2.0 mass%, and further preferably 0.1 to 1.0 mass% with respect to the total amount of the red iron oxide dispersion of red color.

When the content of the acid having a molecular weight of 300 or less is less than 0.01 mass%, the above-described effects become insufficient. When the content is more than 2.0 mass%, the pH decreases, which is not preferable.

The balance of the red iron oxide dispersion for cosmetics is adjusted with water (purified water, distilled water, ion exchanged water, pure water, tap water, etc.).

The red iron oxide dispersion for cosmetics (B) of the present disclosure contains at least the red iron oxide, an organic acid having a molecular weight of 300 or less, and water, and can be produced by blending these components in the ranges of the contents, and uniformly stirring and mixing them.

For example, the red iron oxide dispersion for cosmetics can be prepared by stirring the red iron oxide, an organic acid having a molecular weight of 300 or less, and a solvent such as water with a general-purpose disperser or the like until they are uniformly mixed, further stirring the mixture with a disperser or the like such as a homomixer until the mixture is uniformly mixed, or mixing and dispersing the mixture with various stirrers or dispersion machines such as an ultrasonic disperser, a planetary mixer, a three roll mill, a ball mill, a bead mill, and a jet mill, in addition to a disperser.

In the red iron oxide dispersion for cosmetics (B) used in the present disclosure, the average particle size of the red iron oxide in a dispersed state is preferably 100 nm or less, and more preferably 10 to 50 nm as determined by cumulant method analysis in a scattering intensity distribution.

Setting the average particle size of the red iron oxide to be 100 nm or smaller can exhibit further stable dispersion and vivid color development. The lower limit of the average particle size is preferably as low as possible but is preferably 10 nm or greater in view of productivity and cost.

### Green or dark blue liquid cosmetic

The green liquid cosmetic of the present disclosure includes the Prussian blue dispersion for cosmetics having the properties described above and the red iron oxide dispersion for cosmetics described above. Preferable examples of applications of the green liquid cosmetic include skincare cosmetics, makeup cosmetics, and nail cosmetic products, which include: makeup cosmetics such as foundations, eyeshadows, eyeliners, eyebrow pencils, mascaras, blushes, nail colors, nail treatments, various gel nails, and lipsticks; and hair colors. Examples of the compositions for body markers include body paints. In addition, the form of the product is not particularly limited, but it can be applied to aqueous products such as liquids, emulsions, creams, pastes, gels, mousses, and sprays, because it is a dispersion liquid (aqueous). In particular, the green liquid cosmetic of the present invention is preferably used for aqueous liquid cosmetics such as eye makeup cosmetics including eyeshadows, eyeliners, eyebrow pencils, and mascaras, and aqueous nail colors because of its dispersion properties.

The green liquid cosmetic of the present disclosure includes the above-described Prussian blue dispersion for cosmetics (A) and the red iron oxide dispersion for cosmetics (B), and in addition to each of the above-described dispersions, a dispersant, a film-forming resin, water serving as a dispersion medium (including purified water, distilled water, ion-exchanged water, pure water, ultrapure water, and the like) and the like can be contained in an eye makeup cosmetic. The content of water is the balance of the above-described components and optional components described below.

The dispersant to be used is to further improve the dispersibility of Prussian blue and red iron oxide, which are coloring materials, and may contain, for example, one or more selected from polyaspartic acid, polyglutamic acid, a styrene-acrylic acid copolymer, a styrene-methacrylic acid copolymer, or a styrene-**α**-methylstyrene-acrylic acid copolymer, a homopolymer or a copolymer containing one or two or more compounds selected from acrylic acid, methacrylic acid, or alkyl esters thereof as raw material monomers, or one or more salts thereof. Examples of the salts include anionic polymer dispersants such as alkali salts such as sodium, potassium, and lithium, ammonium salts, and alkanolamine salts such as mono-, di-, triethanolamine, and triisopropanolamine.

Of these dispersants, it is particularly preferable to use at least one type or more of polyaspartic acid, polyglutamic acid, a styrene-acrylic acid copolymer, a styrene-methacrylic acid copolymer, a styrene-**α**-methylstyrene-acrylic acid copolymer, a copolymer of a mixture of tert-butyl acrylate, ethyl acrylate, and methacrylic acid, or salts thereof because the dispersion stability is further excellent, and sodium polyaspartate is particularly preferable.

The content of these dispersants is preferably 0.1 to 10.0%, and more preferably 0.2 to 5.0% with respect to the total amount of the green liquid cosmetic from the viewpoint of achieving both dispersibility of Prussian blue and red iron oxide as coloring materials and dispersion stability in a suitable viscosity range at high degree.

Examples of the film-forming resin that can be used include aqueous emulsion resins of copolymers selected from one or two or more monomers of acrylic acid, methacrylic acid, or alkyl esters or derivatives thereof, styrene, and vinyl acetate.

The content of the film-forming resin (aqueous emulsion resin) is preferably 2 to 15%, more preferably 2 to 10% in terms of solid content (resin content) with respect to the total amount of the green liquid cosmetic from the viewpoints of water resistance performance, coating performance, and the like.

Further, the green liquid cosmetic of the present disclosure can contain, in addition to the above-described components, optional components used in ordinary liquid cosmetics, and the like, as in the case of the cosmetic using only the above-described Prussian blue dispersion for cosmetics (A). Specifically, preservatives, antioxidants, neutralizing agents, ultraviolet absorbers, chelating agents, humectants such as 1,3-butylene glycol, beauty ingredients, perfumes, viscosity/stickiness modifiers, other dispersants such as polyethylene glycol alkyl ethers, and the like can be contained in appropriate amounts within a range that does not impair the effect of the present invention.

The green liquid cosmetic of the present disclosure preferably has a viscosity of 10 mPa·s or less, more preferably 2 to 8 mPa·s, as in the case of the cosmetic using only the above-described Prussian blue dispersion for cosmetics (A) . When the viscosity exceeds 10 mPa·s, the outflow property from an applicator may be remarkably lowered, which is not preferable.

The average particle size in the green liquid cosmetic of the present disclosure is preferably 80 to 250 nm, and more preferably 100 to 230 nm from the viewpoint of further improving coating properties and stability over time.

The viscosity range and the average particle size can be adjusted by suitably combining the ingredients of the above-described Prussian blue dispersion (A), the ingredients of the red iron oxide dispersion of red color (B), and the raw materials such as a dispersant, and combining their contents in suitable ranges, or by a suitable dispersion method. For example, the green liquid cosmetic is obtained by adjusting the dispersion conditions to be suitable by use of a dispersion machine such as a homomixer, a sand mill, an ultrasonic homogenizer, or a high-pressure homogenizer.

Preferably, when a (multiple type) ultrasonic homogenizer or a high-pressure homogenizer is used as the dispersion machine, the dispersion can be prepared by setting the dispersion conditions to, for example, a frequency of 20 to 30 KHz in the case of an ultrasonic homogenizer, and a pressure of 150 to 245 MPa in the case of a high-pressure homogenizer.

When the cosmetic including the above-described Prussian blue dispersion for cosmetics of the present disclosure and the green liquid cosmetic of the present disclosure configured as described above are used, a general-purpose applicator used for eye makeup cosmetics or the like can be used, and the shape, structure, and the like of the applicator to be used are not particularly limited. Examples thereof include an applicator including a knock-type valve device, an applicator including a padding type porous channel having capillary force and an applicator portion, an applicator including a brush applicator portion, a direct liquid type applicator equipped with a collector function, a tube type applicator, and an applicator including a piston pressing mechanism.

The cosmetic containing the Prussian blue dispersion for cosmetics of the present embodiment constituted as described above, which contains a pigment dispersion for cosmetics containing at least the Prussian blue, the water-soluble basic substance in an amount of 0.025 to 0.25 in terms of mass ratio when the content of the Prussian blue is defined as 1 in terms of mass ratio, water, and, for example, a film-forming agent, a pH modifier, a dispersant (stabilizer), and a preservative, is excellent in dispersion stability, color-developing properties (high chroma), and storage stability of Prussian blue, wherein Prussian blue used for cosmetics can be stably dispersed.A cosmetic excellent in dispersion stability, storage stability, usability, coating properties, and the like can be obtained.

Further, the green liquid cosmetic of the present disclosure, contains the above-described Prussian blue dispersion for cosmetics (A) containing at least Prussian blue, a water-soluble basic substance, and water, and an amount of the water-soluble basic substance is 0.05 to 0.13 by mass ratio when the content of the Prussian blue is defined as 1 by mass ratio; and the red iron oxide dispersion for cosmetics (B) containing a red iron oxide of red color, an organic acid having a molecular weight of 300 or less, and water, wherein the red iron oxide has an average particle size of 200 nm, preferably 100 nm or less as determined by cumulant method analysis in a scattering intensity distribution. Thus, the green liquid cosmetic is excellent in dispersion stability, color-developing properties (high chroma), and weather resistance. Then, with color mixing with the red iron oxide dispersion of red color, the green liquid cosmetic which is excellent in green color development (high chroma), dispersion stability, weather resistance, and coating properties, and suitable for makeup cosmetics such as eyeshadows, eyeliners, eyebrow pencil, mascaras, and the like, can be obtained.

### Examples

Next, the present disclosure will be described in further detail with reference to examples and comparative examples, although the present disclosure is not limited to the following examples and the like.

### Prussian blue dispersion for cosmetics: Examples 1 to 9 and Comparative Examples 1 to 4

Prussian blue dispersions for cosmetics were prepared in the formulations tabulated in the following Table 1 and by the dispersion after the following method.

For the obtained Prussian blue dispersions for cosmetics, the viscosity, pH and average particle size of the dispersions were evaluated by the following methods, and the color-developing properties and storage stability of a cosmetic using each of the Prussian blue dispersions for cosmetics was evaluated by the following evaluation methods.

The results are shown in Table 1 below.

### Dispersion method

Prussian blue, a basic substance, water, and optionally a dispersant were stirred and mixed by using a general-purpose disperser in the formulations shown in Table 1 below, and the mixed liquid was caused to pass through a bead mill disperser (LMZ015: available from Ashizawa Finetech Ltd.), and dispersed while adjusting dispersion conditions (peripheral speed) with observation of the dispersion state.

In Dispersion condition (peripheral speed) in the table, "medium" is 6 to 10 m/s, the dispersion time is 30 to 300 minutes, and "strong" is a condition of "medium" or more.

### Method for measuring pH

The pH of each of the Prussian blue liquids for cosmetics before dispersion and each of the Prussian blue dispersion for cosmetics obtained by the following dispersion method was measured at 25°C using a glass electrode pH meter.

### Method for measuring viscosity

The viscosity of each Prussian blue dispersions for cosmetics obtained by the above method was measured at 25°C with a cone-plate type viscosimeter (TVE-25, available from Toki Sangyo Co., Ltd.) under the condition of a shear rate of 383 s⁻¹.

### (Method for measuring average particle size)

The average particle size was measured by a dynamic light scattering method using FPAR-1000 (available from Otsuka Electronics Co., Ltd.).

### Evaluation method for color-developing properties

A cosmetic was prepared by adding a solvent (1,3-butylene glycol), a pH modifier (2-amino-2-methyl-1-propanol), a preservative (phenoxyether, parabens), a film-forming resin (aqueous emulsion), and water (purified water) to each of the obtained Prussian blue dispersions for cosmetics. The cosmetic was filled in an UC-76B container available from Mitsubishi Pencil Company, Limited and evaluated for color-developing properties according to the following evaluation criteria.

Evaluation criteria:
Good: The discharge property from the container is good, and good color development is achieved.
Medium: The discharging property from the container is not good , and the color development is not good.
Poor: The discharging property from the container is much worce, and the color development was poor.

### Method for evaluating storage stability

For each of the obtained Prussian blue dispersions for cosmetics, a cosmetic was prepared in the same manner as in the above-described evaluation of color-developing properties and filled in an UC-76B container available from Mitsubishi Pencil Company, Limited to evaluate storage stability according to the following evaluation criteria. Evaluation criteria for storage stability:
Good: Sedimentation of the pigment in storage over time is small, and the difference in concentration from the initial state is not large.
Medium: Sedimentation of the pigment was observed to considerable extent in storage over time, and the difference in concentration from the initial time was large.
Poor: Discharge of the cosmetic is poor, and usability is poor.

**[Table 1]**

| | | (Total amount: 100 mass% each) | | | | | | | | | | | | |
|---|---|---|---|---|---|---|---|---|---|---|---|---|---|---|
| | | Examples | | | | | | | | | Comparative Examples | | | |
| | | 1 | 2 | 3 | 4 | 5 | 6 | 7 | 8 | 9 | 1 | 2 | 3 | 4 |
| Prussian blue *1 | | 20.000 | 20.000 | 20.000 | 20.000 | 10.000 | 50.000 | 20.000 | 20.000 | 20.000 | 20.000 | 30.000 | 40.000 | 20.000 |
| Basic substance A *5 | | 1.000 | 1.500 | 2.000 | 1.000 | 0.750 | 3.750 | 5.000 | | | | | | |
| Basic substance B *6 | | | | | | | | | 0.500 | 1.500 | | | | |
| Dispersant *4 | | 0.075 | 0.075 | 0.075 | | | | | | | | | | 0.075 |
| Water (purified water) | | 78.925 | 78.425 | 77.925 | 79.000 | 89.250 | 46.250 | 75.000 | 79.500 | 78.500 | 80.000 | 70.000 | 60.000 | 79.925 |
| Before dispersion | pH | 10.3 | 10.5 | 10.8 | 10.4 | 9.8 | 12.1 | 12 | 9.8 | 11.3 | 6.8 | 6.8 | 6.8 | 6.8 |
| Dispersion conditions | Peripheral speed | Medium | Medium | Medium | Medium | Medium | Medium | Medium | Medium | Medium | Medium | Medium | Medium | Strong |
| Dispersion physical properties | Viscosity (25 ° C: mPa·s) | 2.2 | 5.1 | 8.1 | 2.2 | 2.1 | 40 | 50 | 2.5 | 5.6 | 2.8 | 3.1 | Thickened | Thickened |
| | pH | 6.5 | 6.6 | 6.7 | 6.2 | 6.4 | 6.9 | 7 | 6.8 | 7.1 | 5.7 | 5.6 | - | - |
| | Average particle size (nm) | 80 to 120 | 80 to 110 | 80 to 120 | 80 to 100 | 80 to 150 | 80 to 150 | 80 to 140 | 80 to 130 | 80 to 130 | 80 to 150 | 80 to 160 | 150 to 250 | 150 to 250 |
| | | | | | | | | | | | | | | |

| Formulation of cosmetic (blending the above dispersion with the following cosmetic components) | | | | | | | | | | | | | | |
|---|---|---|---|---|---|---|---|---|---|---|---|---|---|---|
| Above-described dispersion | | 40 | 40 | 40 | 40 | 40 | 40 | 40 | 40 | 40 | 40 | 40 | 40 | 40 |
| 1,3-butylene glycol (BG) | | 8 | 8 | 8 | 8 | 8 | 8 | 8 | 8 | 8 | 8 | 8 | 8 | 8 |
| 2-amino-2-methyl-1-propanol | | 0.2 | 0.2 | 0.2 | 0.2 | 0.2 | 0.2 | 0.2 | 0.2 | 0.2 | 0.2 | 0.2 | 0.2 | 0.2 |
| Film-forming resin A *2 | | 24 | 24 | 24 | 24 | 24 | 24 | 24 | 24 | 24 | 24 | 24 | 24 | 24 |
| Preservative *3 | | 0.9 | 0.9 | 0.9 | 0.9 | 0.9 | 0.9 | 0.9 | 0.9 | 0.9 | 0.9 | 0.9 | 0.9 | 0.9 |
| Water (purified water) | | 26.9 | 26.9 | 26.9 | 26.9 | 26.9 | 26.9 | 26.9 | 26.9 | 26.9 | 26.9 | 26.9 | 26.9 | 26.9 |
| Dispersion evaluation | Color-developing properties | Good | Good | Good | Good | Good | Good | Medium | Good | Good | Good | Good | Poor | Poor |
| | Storage stability | Good | Good | Good | Good | Medium | Medium | Medium | Good | Good | Poor | Poor | Poor | Poor |
| *1: IRON BLUE SC, available from Daito Chemical Industry Co., Ltd. | | | | | | | | | | | | | | |
| *2: an aqueous emulsion 1; DAITOSOL 5000PO (solids content 50%, average particle size 130 nm) | | | | | | | | | | | | | | |
| *3: a mixture of methylparaben, ethylparaben, and phenoxyethanol | | | | | | | | | | | | | | |
| *4: a nonionic surfactant, ceteth-20, available from Nikko Chemicals Co., Ltd. | | | | | | | | | | | | | | |
| *5: 2-amino-2-methyl-1-propanol | | | | | | | | | | | | | | |
| *6: potassium hydroxide | | | | | | | | | | | | | | |

From the results in Table 1 above, it was found that Examples 1 to 9, which fall within the support range of the Prussian blue dispersion for cosmetics of the present disclosure, exhibited excellent color-developing properties and storage stability as compared with Comparative Examples 1 to 4, which were outside the scope of the present disclosure.

Comparative Examples 1 to 4, which were outside the scope of the present disclosure, did not contain a water-soluble basic substance, and only Comparative Example 4 further contained a dispersant. Comparative Examples 1 and 2 were inferior in storage stability, and Comparative Examples 3 and 4 were thickened (gelled) and could not be formed into fine particles.

According to the results of Examples 1 to 9, it was surprisingly confirmed that the dispersion state was stable even when the pH-value of the dispersion was made basic, the average particle size itself was able to be reduced to about 100 nm to 200 nm (preferably 130 nm or less), and the color-developing properties and storage stability were excellent.

In addition, when each of the cosmetics obtained in Examples 1 to 4, 7, and 9 was accommodated in a cosmetic applicator (applicator configuration: applicator portion; a brush, equipped with a collector function, UC-76C; available from Mitsubishi Pencil Company, Limited) and applied to an eyelid, it was confirmed that, despite the low viscosity, no bleeding occurred and despite the fact that an extremely thin line was able to be drawn, a vivid blue and clear line was drawn.

### Production Examples 1 to 7

Red iron oxide dispersions A to C were produced (prepared) in the following Production Examples 1 to 3 by using the Prussian blue dispersion of Examples 1, 3, and 4 and Comparative Example 4 as A to D, respectively. The pH, viscosity, and average particle size of each of the obtained Prussian blue dispersions A to D of Examples 1, 3, and 4 and Comparative Example 4 and the red iron oxide dispersions of red color A to C of Production Examples 1 to 3 were measured by the following methods.

### Method for measuring pH

The pH of each obtained dispersion was measured at 25°C with a glass electrode pH meter.

### Method for measuring viscosity

The viscosity of each obtained dispersion was measured at 25°C using a cone-plate type viscosimeter (TVE-25, available from Toki Sangyo Co., Ltd.) under the condition of a shear rate of 383 s⁻¹.

### (Method for measuring average particle size)

The average particle size of particles in each dispersion liquid (25°C) was measured by a dynamic light scattering method using FPAR-1000 (available from Otsuka Electronics Co., Ltd.).

### Production Example 1: Preparation of Red Iron Oxide Dispersion of Red Color A

Red iron oxide (No. 216P, available from Daito Chemical Industry Co., Ltd.) 20.0%
Dispersant: sodium polyaspartate 3.0%
Citric acid 1.50%
Water (purified water) Balance

The dispersion was prepared using the above components with a bead mill disperser.
pH: 7.5, Viscosity: 2.5 mP·s, Average particle size: 20 to 70 nm

### Production Example 2: Preparation of Red Iron Oxide Dispersion of Red Color B

Red iron oxide (No. 216P, available from Daito Chemical Industry Co., Ltd.) 20.000%
Dispersant: sodium polyaspartate 8.667%
Citric acid 0.667%
Water (purified water) Balance

The dispersion was prepared using the above components with a bead mill disperser.
pH: 7.5, Viscosity: 3.0 mP·s, Average particle size: 20 to 70 nm

### Production Example 3: Preparation of Red Iron Oxide Dispersion of Red Color C

Red iron oxide (No. 216P, available from Daito Chemical Industry Co., Ltd.) 20.000%
Dispersant: sodium polyaspartate 3.000%
Water (purified water) Balance

The dispersion was prepared using the above components with a bead mill disperser.
pH: 8.6, Viscosity: 7.5 mP·s, Average particle size: 150 to 300 nm

### Example 10 to 14 and Reference Examples 1 to 4

Green liquid cosmetics were prepared using the above-described Prussian blue dispersions A to D and the red iron oxide dispersions A to C of Production Examples 1 to 3 according to the formulation shown in the following Table 2 and dispersion by the following method.

The obtained green liquid cosmetics were evaluated for average particle size (after preparation, after 3 months (3M) at 25°C), pH (after preparation, after 3 months (3M) at 25°C), vertical concentration difference after 3 months (3M) at 25°C, coating properties, and color-developing properties.

These results are indicated in Table 2 below.

### Dispersion method

Dispersion was performed with the same bead mill disperser as described above using Zirconia 0.3 mm as beads.

### Method for measuring pH

The pH of each of the obtained green liquid cosmetics was measured at 25°C (after 3 months (3M) at 25°C, after preparation) using a glass-electrode pH-meter.

### Method for measuring viscosity

The viscosity of each of the obtained green liquid cosmetics was measured at 25°C using a cone-plate type viscosimeter (TVE-25, available from Toki Sangyo Co., Ltd.) under the condition of a shear rate 383 s⁻¹ (after 3 months (3M) at 25°C, after preparation).

### (Method for measuring average particle size)

The average particle size (after 3 months (3M) at 25°C, after preparation) of each of the obtained green liquid cosmetics was measured by a dynamic light scattering method using FPAR-1000 (available from Otsuka Electronics Co., Ltd.).

### Method for evaluating vertical concentration difference

Each green liquid cosmetic was filled in a direct liquid type eyeliner available from Mitsubishi Pencil Company, Limited (application body: UC-76B, tuft type), and sensory evaluation was performed according to the following evaluation criteria by observing the state of drawn lines after 3 months at room temperature (25°C) with the application body facing upward and downward.

### Evaluation criteria:

Good: Vertical concentration difference is small
Medium: There is a Vertical concentration difference, but there is no change in hue.
Poor: Vertical concentration difference and hue difference are clearly observed.

### Method for evaluating coating property

Each obtained green liquid cosmetic was filled in an UC-76B container available from Mitsubishi Pencil Company, Limited and applied to a part around an eye, and the coating properties after a lapse of time (after 3M at 25°C) was evaluated according to the following evaluation criteria. Evaluation criteria:
Good: Coating can be performed without blurring.
Medium: Coating is possible although slight blurring is observed. Poor: Coating cannot be performed because of blurring.

### Evaluation method for color-developing properties

Using each obtained green liquid cosmetic, a film was coated on Katherine paper (available from Tokushu Tokai Paper Co., Ltd.) with a bar coater after a lapse of time (after 3M at 25°C) . The hue (a*, b*) was evaluated with a spectrophotometer (available from Suga Test Instruments Co., Ltd., SC-P) .

**[Table 2]**

| | | | | | | | (Total amount: 100 mass%) | | |
|---|---|---|---|---|---|---|---|---|---|
| | Examples | | | | | Reference Example | | | |
| | 10 | 11 | 12 | 13 | 14 | 1 | 2 | 3 | 4 |
| Example 1: Prussian blue dispersion A (average particle size 80 to 120 nm) | 14.5 | | 14.5 | 14.5 | | | | | 14.5 |
| Example 3: Prussian blue dispersion B (average particle size 80 to 120 nm) | | 14.5 | | | | | 14.5 | | |
| Example 4: Prussian blue dispersion C (average particle size 80 to 100 nm) | | | | | 14.5 | | | | |
| Comparative Example **4:** Prussian blue dispersion D (average particle size 150 to 250 nm) | | | | | | 14.5 | | 14.5 | |
| Production Example **1:** Red iron oxide dispersion A (average particle size 20 to 70 nm) | 30.5 | 30.5 | 30.5 | | 30.5 | 30.5 | 30.5 | 30.5 | |
| Production Example 2: Red iron oxide dispersion B (average particle size 20 to 70 nm) | | | | 30.5 | | | | | |
| Production Example 3: Red iron oxide dispersion C (average particle size 150 to 300 nm) | | | | | | | | | 30.5 |
| Film-forming resin A (aqueous emulsion 1) *1 | 20 | 20 | | 20 | 20 | 20 | 20 | | |
| Film-forming resin B (aqueous emulsion 2) *2 | | | 24 | | | | | 24 | 24 |
| Triethanolamine | | | | | | 0.1 | 0.1 | | |
| 1,3-butylene glycol | 10 | 10 | 10 | 10 | 10 | 10 | 10 | 8 | 8 |
| Preservative *3 | 0.8 | 0.8 | 0.8 | 0.8 | 0.8 | 0.8 | 0.8 | 1 | 1 |
| Water (purified water) | Balance | Balance | Balance | Balance | Balance | Balance | Balance | Balance | Balance |
| Average particle size (nm) | 220 | 220 | 120 | 210 | 220 | 220 | 240 | 110 | 120 |
| Average particle size (nm) (after 3M at 25°C) | 200 | 230 | 110 | 220 | 220 | 220 | 230 | 100 | 130 |
| pH (25°C) | 6.9 | 8.0 | 7.1 | 6.9 | 6.5 | 7.1 | 7.6 | 7.2 | 7.1 |
| pH (after 3M at 25°C) | 6.8 | 7.0 | 6.9 | 6.8 | 6.4 | 6.7 | 6.7 | 6.8 | 6.8 |
| Viscosity | 3.1 | 3.1 | 2.7 | 3.0 | 3.0 | 3.0 | 3.0 | 2.5 | 2.5 |
| Viscosity (after 3M at 25°C) | 2.9 | 2.9 | 3.2 | 3.0 | 3.0 | 7.0 | 7.2 | 2.6 | 2.3 |
| Vertical hue difference in product after a lapse of time (after 3M at 25°C) (whether green color is maintained over time) | Good | Good | Good | Good | Good | Poor | Poor | Poor | Poor |
| | | | | | | Thickened | Thickened | Prussian blue settled | Red iron oxide settled |
| Coating property after a lapse of time (after 3M at 25°C) | Good | Good | Good | Good | Good | Medium | Medium | Good | Good |
| Color-developing property after a lapse of time (after 3M at 25°C): a* | -15 | -15 | -15 | -15 | -15 | -14 | -14 | -3 | 22 |
| Color-developing property after a lapse of time (after 3 M at 25°C): b* | 20 | 20 | 20 | 20 | 20 | 21 | 21 | -5 | 26 |
| ***1: solid content 57% (average particle size 240 nm)** | | | | | | | | | |
| ***2: solid content 45% (average particle size 100 nm)** | | | | | | | | | |
| ***3: a mixture of methylparaben, ethylparaben, phenoxyethanol, and ethylhexylglycerin** | | | | | | | | | |

From the results shown in Table 2 above, it was found that Examples 10 to 14, which fall within the support range of the green liquid cosmetic of the present disclosure, exhibited excellent vertical concentration difference, coating property, color-developing properties, and stability of viscosity after a lapse of 3 months at 25°C as compared with Reference Examples 1 to 4, which were outside the support range of the green liquid cosmetic of the present disclosure.

In Examples 10 to 14 which fall in the scope of the present disclosure, the apparent average particle size was stable as 210 to 220 nm and was maintained as 200 to 220 nm even after 3M at room temperature (25°C), and the pH was also stable. The hue after a lapse of 3M was also maintained green (a* = -15, b* = 20).

On the other hand, in Reference Examples 1 and 2, the content of the water-soluble base substance was outside the scope of the present disclosure, and they had the results probably because of unstable dispersed state of red iron oxide. In Reference Example 3 in which the average particle size of Prussian blue is large, the Prussian blue precipitated. On the other hand, in Reference Example 4 in which a red iron oxide dispersion having an average particle size of 150 to 300 nm was used, the red iron oxide precipitated.

Surprisingly, in the range of the green liquid cosmetic of the present disclosure (Examples 10 to 14), a liquid cosmetic (eyeliner) that develops green color was able to be formed by using a Prussian blue dispersion having basic pH whose average particle size was made finer to 130 nm or less and combining the dispersion with a red iron oxide dispersion of red color containing an organic acid having a molecular weight of 300 or less. Further, by mixing with a fine dispersion (30 to 100 nm) of a red iron oxide of red color, an eyeliner whose hue appears green even after a lapse of time was able to be obtained.

### Industrial Applicability

A Prussian blue dispersion for cosmetics excellent in dispersion stability, color-developing properties(high chroma), and storage stability can be obtained, and by using this Prussian blue dispersion, a cosmetic suitable for base makeup cosmetics such as foundations and undermakeups, and point makeup cosmetics such as eyeshadows, eyeliners, mascaras, eyebrow pencils, and nail colors excellent in color-developing properties (high chroma) and storage stability, which was not known before, can be obtained.

In addition, a green liquid cosmetic suitable for makeup cosmetics such as eyeshadows, eyeliners, eyebrow pencils, mascaras, and the like, and a composition for body markers suitable for body paints and the like, which are excellent in green color-developing properties (high chroma), dispersion stability, and weather resistance, can be obtained by blending the Prussian blue dispersion for cosmetics and the red iron oxide dispersion of red color having the specific properties.

## Claims

1. A Prussian blue dispersion for cosmetics, the Prussian blue dispersion comprising at least Prussian blue, a water-soluble basic substance, and water.

2. The Prussian blue dispersion for cosmetics according to claim 1, the Prussian blue dispersion containing the water-soluble basic substance in a range of 0.025 to 0.25 when a content of the Prussian blue is defined as 1 in terms of mass ratio.

3. The Prussian blue dispersion for cosmetics according to claim 1 or 2, wherein the water-soluble basic substance is at least one type selected from ammonia, a compound having an amino group, or an alkali metal hydroxide.

4. The Prussian blue dispersion for cosmetics according to any one of claims 1 to 3, the Prussian blue dispersion having a viscosity of 50 mPa·s or less at a shear rate of 383 s⁻¹ in viscosity measurement at 25°C with a cone-plate type viscosimeter.

5. The Prussian blue dispersion for cosmetics according to any one of claims 1 to 4, wherein the Prussian blue has an average particle size of 200 nm or less as determined by cumulant method analysis in a scattering intensity distribution.

6. The Prussian blue dispersion for cosmetics according to any one of claims 1 to 5, further comprising a dispersant.

7. A cosmetic formed by blending the Prussian blue dispersion for cosmetics described in any one of claims 1 to 6.

8. The cosmetic according to claim 7, further comprising an iron oxide pigment dispersion.

9. A green liquid cosmetic comprising at least a Prussian blue dispersion for cosmetics and an iron oxide pigment dispersion, wherein the Prussian blue dispersion for cosmetics is a Prussian blue dispersion for cosmetics (A) containing at least Prussian blue, a water-soluble basic substance, and water, and an amount of the water-soluble basic substance is 0.05 to 0.13 when a content of the Prussian blue is defined as 1 in terms of mass ratio; the iron oxide pigment dispersion is a red iron oxide dispersion for cosmetics (B) containing red iron oxide, an organic acid having a molecular weight of 300 or less, and water, and the red iron oxide having an average particle size of 100 nm or less as determined by cumulant method analysis in a scattering intensity distribution.

10. The green liquid cosmetic according to claim 9, wherein the Prussian blue in the Prussian blue dispersion for cosmetics (A) has an average particle size of 130 nm or less as determined by cumulant method analysis in a scattering intensity distribution.

11. The green liquid cosmetic according to claim 9 or 10, wherein the organic acid having a molecular weight of 300 or less in the red iron oxide dispersion for cosmetics (B) is selected from Group A below:
Group A: glycolic acid, lactic acid, tartronic acid, glyceric acid, hydroxybutyric acid, 2-hydroxybutyric acid, 3-hydroxybutyric acid, γ-hydroxybutyric acid, malic acid, tartaric acid, citramalic acid, citric acid, isocitric acid, leucinic acid, mevalonic acid, pantoic acid, ricinoleic acid, ricinelaidic acid, cerebronic acid, quinic acid, shikimic acid.

12. The green liquid cosmetic according to claim 11, wherein the organic acid having a molecular weight of 300 or less in the red iron oxide dispersion for cosmetics (B) is citric acid.
